## Europäisches Patentamt
## European Patent Office
## Office européen des brevets

(11) Veröffentlichungsnummer: **0 144 721**
**A1**

(12) **EUROPÄISCHE PATENTANMELDUNG**

(21) Anmeldenummer: 84113125.3

(22) Anmeldetag: 31.10.84

(51) Int. Cl.⁴: **A 01 N 57/16**

(30) Priorität: 12.11.83 DE 3341017

(43) Veröffentlichungstag der Anmeldung:
19.06.85 Patentblatt 85/25

(84) Benannte Vertragsstaaten:
AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Toshkov, Alexander Stoyanov, Prof.
Praga Strasse 24
Sofia 1618(BG)

(72) Erfinder: Schabanov, Mehmed Mustafov, Prof. Dr.
"Ljulin", Strasse 411 Block 429
Sofia 1336(BG)

(72) Erfinder: Nedjalkov, Stoiko Stoikov, Prof. Dr.
Boris I Strasse 32
Sofia 1000(BG)

(54) **Varoatosemittel.**

(57) Die Erfindung betrifft 0,0-Diethyl-0-(3-chlor-4-methyl-cumarin-7-yl)-thionophosphorsäureester zur Bekämpfung von Varoa Jacobsoni auf Bienen, 0,0-Diethyl-0-(3-chlor-4-methyl-cumarin-7-yl)-thionophosphorsäureester enthaltende Varoatosemittel, Herstellungsverfahren für diese Mittel und die Verwendung von 0,0-Diethyl-0-(3-chlor-4-methyl-cumarin-7-yl)-thionophosphorsäureester zur Bekämpfung von Varoa Jacobsoni.

EP 0 144 721 A1

BAYER AKTIENGESELLSCHAFT          5090 Leverkusen, Bayerwerk

Konzernverwaltung RP              Ad/Brü
Patentabteilung


Varoatosemittel


Die Erfindung betrifft O,O-Diethyl-O-(3-chlor-4-methyl-cumarin-7-yl)-thionophosphorsäureester zur Bekämpfung von Varoa Jacobsoni auf Bienen, O,O-Diethyl-O-(3-chlor-4-methyl-cumarin-7-yl)-thionophosphorsäureester enthaltende Varoatosemittel, Herstellungsverfahren für diese Mittel und die Verwendung von O,O-Diethyl-O-(3-chlor-4-methyl-cumarin-7-yl)-thionophosphorsäureester zur Bekämpfung von Varoa Jacobsoni.

Die Varoatose wird von der Milbe Varoa Jacobsoni hervorgerufen, die ausgewachsen auf Bienen, Drohnen und Bienenköniginnen und in ihren präimaginalen Entwicklungsformen auf Bienen- und Drohnenlarven und den Puppen parasitiert.

Dieser Parasitismus ist neu in der Bienenzucht und erreicht bereits eine panzootische Form. Durch akute Schwächung und hohe Sterblichkeit der Bienenvölker ruft die Varoatose große Schäden in der Bienenzucht hervor. Auch die unmittelbaren bienenzüchterischen Produkte wie Honig, Wachs, Bienenmilch, Bienengift sind davon betroffen. Auf Grund der Erniedrigung der Bestäubung der entomophilen landwirtschaftlichen Kulturen beeinträchtigt sie

Le A 22 737

aber auch den mittelbaren Nutzen der Bienen, der 10 - 15fach die unmittelbare Produktion der Bienen übersteigt.

Bisher sind Methoden zur Bekämpfung der Varoatose bei Bienen bekannt, bei denen die Bienenvölker abgeraucht oder mit chemischen Wirkstoffen bestäubt werden.

Bekannt ist auch die Methode der Thermotherapie und der mechanischen Beseitigung der abgedeckten Drohnenbrut.

Der Nachteil der bekannten Methoden ist, daß sie nicht sehr effektiv sind, auch nicht bei mehrfacher Anwendung mit für die Bienenköniginnen und Drohenbrut verträglichen Dosierungen. Einige bislang verwendete Mittel sind schädlich und giftig für die Bienen, die Brut und die Königinnen, aber auch für den Anwender. Ein weiterer Nachteil ist der große manuelle Aufwand bei der Anwendung.

Die bisher verwendeten chemischen Wirkstoffe greifen nur erwachsene Milben an, die an den Bienen, Drohnen und Königinnen haften. Dieses erfolgt jedoch nur in unbefriedigendem Maße, und zwar nicht nur bei einmaliger, auch bei mehrmaliger Bearbeitung und Behandlung der parasitenbefallenen Bienenvölker. Außerdem wird bei ihrer Anwendung, die durch Abrauchen, Besprühen oder durch Bestäubung erfolgt, ein Teil dieser Wirkstoffe von den die kranken Bienenvölker betreuenden Imkern eingeatmet, so daß es nicht selten zu schweren Vergiftungen kommt.

Le A 22 737

Es ist Aufgabe der Erfindung, eine Methode zur Bekämpfung der Varoatose bei Bienen zu schaffen, die effektiv und unschädlich sowohl für die Bienen, die Brut und die Königinnen, als auch für den Imker leicht anwendbar ist.

Diese Aufgabe wird gelöst durch Verwendung von O,O-Diethyl-O-(3-chlor-4-methyl-cumarin-7-yl)-thiono-phosphorsäureester zur Bekämpfung der Varoatose.

Gegenstand der Erfindung ist daher O,O-Diethyl-O-(3-chlor-4-methyl-cumarin-7-yl)-thionophosphorsäureester zur Bekämpfung der Varoatose bzw. von Varoa Jacobsoni auf Bienen.

O,O-Diethyl-O-(3-chlor-4-methyl-cumarin-7-yl)-thiono-phosphorsäureester sowohl zur Bekämpfung von Varoa Jacobsoni auf den Bienen als auch zur Bekämpfung der Entwicklungsformen von Varoa Jacobsoni auf Bienen- und Drohnenlarven und Puppen.

Weiterer Gegenstand der Erfindung sind Mittel zur Bekämpfung von Varoa Jacobsoni und deren Entwicklungsstadien auf Bienen, enthaltend O,O-Diethyl-O-(3-chlor-4-methyl-cumarin-7-yl)-thionophosphorsäureester, Trägersubstanzen und gegebenenfalls Formulierungshilfsmittel.

Insbesondere enthalten die erfindungsgemäßen Mittel in der Anwendungsform 0,0000001 bis 95 Gew.-% O,O-Diethyl-O-(3-chlor-4-methyl-cumarin-7-yl)-thionophosphorsäure-ester, vorzugsweise 0,0001 bis 10 Gew.-%, ganz besonders bevorzugt 0,001 bis 1 Gew.-%.

Als Trägersubstanzen eignen sich bevorzugt Rohrzucker, Glukose oder andere bienenverträgliche Zucker. Die Mittel können den Zucker granuliert, kristallin, sirupös oder als wäßrige Lösung enthalten.

Le A 22 737

Bevorzugt enthalten die Mittel auf 0,5 bis 1,0 Liter Zuckersirup 0,5 bis 0,6 g O,O-Diethyl-O-(3-chlor-4-methyl-cumarin-7-yl)-thionophosphorsäureester.

Neben der Anwendung in einem Sirup durch Verfüttern haben sich die im folgenden beschriebenen Formulierungen des erfindungsgemäß verwendeten Wirkstoffs als besonders vorteilhaft erwiesen:

## 1. Stäubemittel

Zur Herstellung einer zweckmäßigen Wirkstoffzubereitung werden 5 Gewichtsteile Wirkstoff mit 95 Gewichtsteilen eines natürlichen Gesteinsmehles vermengt und staubfein vermahlen. Das so erhaltene Mittel wird in der jeweils gewünschten Menge durch Verstäuben auf die Bienen oder ihren Lebensraum aufgebracht.

## 2. Spritzpulver (Dispergierbares Pulver)

a) Formulierung eines flüssigen Wirkstoffes.
Zur Herstellung einer zweckmäßigen Wirkstoffzubereitung werden 25 Gewichtsteile Wirkstoff mit 1 Gewichtsteil Dibutylnaphthalinsulfonat, 4 Gewichtsteilen Ligninsulfonat, 8 Gewichtsteilen hochdisperser Kieselsäure sowie 62 Gewichtsteilen eines natürlichen Gesteinsmehles vermischt und zu einem Pulver vermahlen. Vor der Anwendung wird das benetzbare Pulver mit soviel Wasser verrührt, daß die dabei entstehende Mischung den Wirkstoff in der jeweils gewünschten Konzentration enthält.

b) Formulierung eines festen Wirkstoffes.
Zur Herstellung einer zweckmäßigen Wirkstoffzubereitung werden 50 Gewichtsteile Wirkstoff mit 1 Gewichtsteil Dibutylnaphthalinsulfonat, 4 Gewichtsteilen Ligninsulfonat, 8 Gewichtsteilen hochdisperser Kieselsäure
sowie 37 Gewichtsteilen eines natürlichen Gesteinsmehles
vermischt und zu einem Pulver vermahlen. Vor der Anwendung wird das benetzbare Pulver mit soviel Wasser
verrührt, daß die dabei entstehende Mischung den Wirkstoff in der jeweils gewünschten Konzentration enthält.


### 3. Emulgierbares Konzentrat

Zur Herstellung einer zweckmäßigen Wirkstoffzubereitung
werden 25 Gewichtsteile Wirkstoff in einer Mischung aus
55 Gewichtsteilen Xylol und 10 Gewichtsteilen Cyclohexanon gelöst. Als Emulgator setzt man anschließend 10 Gewichtsteile einer Mischung auf Dodecylbenzolsulfonsaurem
Calcium und Nonylphenolpolyglykolether hinzu. Vor der Anwendung wird das Emulsionskonzentrat mit soviel Wasser
verdünnt, daß die dabei entstehende Mischung den Wirkstoff
in der jeweils gewünschten Konzentration enthält.


### 4. Granulat

a) Formulierung eines flüssigen Wirkstoffes.
   Zur Herstellung einer zweckmäßigen Wirkstoffzubereitung
   tung wird 1 Gewichtsteil Wirkstoff gemäß Beispiel
   auf 9 Gewichtsteile granulierten saugfähigen Ton aufgesprüht. Das entstehende Granulat wird in der jeweils
   gewünschten Menge auf die Bienen oder deren Lebensraum verstreut.

b) Formulierung eines festen Wirkstoffes.
   Zur Herstellung einer zweckmäßigen Wirkstoffzubereitung
   tung gibt man zu 91 Gewichtsteilen Sand der Körnung
   0,5 - 1,0 mm 2 Gewichtsteile eines Spindelöls und


Le A 22 737

anschließend 7 Gewichtsteile einer feingemahlenen Wirkstoffvermischung, die ihrerseits 75 Gewichtsteile Wirkstoff und 25 Gewichtsteile natürliches Gesteinsmehl enthält. Die Mischung wird so lange in einem geeigneten Mischer behandelt, bis ein gleichmäßiges, frei fließendes und nicht staubendes Granulat entstanden ist. Das Granulat wird in der jeweils gewünschten Menge auf die Bienen oder deren Lebensraum verstreut.

Eine besonders bevorzugte erfindungsgemäße Formulierung enthält 50 g O,O-Diethyl-O-(3-chlor-4-methyl-cumarin-7-yl)-thionophosphorsäureester, 3 g Emulgator 368 (Polyethylenglykolether), 3 g Schutzkolloid 63 (Schiff'sche Base aus Anilin und Ligninsulfonat), 14 g hochdisperse Kieselsäure und ad 100 g kolloidales Kaolin.

Die erfindungsgemäßen Mittel können auch zur Abtötung der Bienenläuse der Gattung Braulau verwendet werden. Auch die Erreger der Akarinose werden vernichtet (Akarapis Woodi).

Die Erfindung betrifft auch Verfahren zur Herstellung der Mittel zur Bekämpfung der Varoatose. Demgemäß wird beispielsweise wie üblich ein Zuckersirup je nach Anzahl und Zustand der Bienenfamilien bereitet. Der Sirup wird auf 35 - 40°C abgekühlt. Der Wirkstoff wird in Wasser suspendiert und dem Sirup zugegeben. Dann wird gut durchgemischt und behandelt. Nach einer Woche wird die Applikation wiederholt. Eine zweimalige Applikation ist günstig im Frühjahr und eine dreimalige im Herbst in Abständen von 7 - 8 Tagen. Der Effekt beträgt mindestens 96 - 97 %.

Le A 22 737

Die Vorteile der erfindungsgemäßen Bekämpfungsmethode bestehen darin, daß sie effektiv, unschädlich für die Brut, die Bienen und die Königinnen und auch für den Anwender ist. Sie ist schnell und nicht arbeitsaufwendig. Sie besitzt eine dauerhafte akarizide Wirkung für 7 bis 8 Tage. Die Anwendung kann mit Beifütterung für die Bienenvölker kombiniert werden.

Mit den erfindungsgemäßen Bekämpfungsverfahren werden nicht nur die adulten Milben, sondern auch ihre Entwicklungsformen in der Brut vernichtet. Die Mittel dringen in die Brut ein, bevor sie abgedeckt ist. Mittels dieser Methode kann ein Imker in einer Stunde 100 und mehr parasitenbefallene Bienenvölker bearbeiten.

Die Erfindung wird anhand des folgenden Ausführungsbeispiels erklärt. Die folgende Tabelle enthält auch Vergleichsdaten bekannter Mittel zur Bekämpfung der Varoatose.

Beispiel:

In einen Liter auf Zimmertemperatur abgekühlten Zuckersirup zur Befütterung der Bienen wird die Dosis von 0,5 g der Verbindung O,O-Diethyl-O-(3-chlor-4-methyl-cumarin-7-yl)-thionophosphorsäureester gut eingerührt. Mit diesem Sirup wird das Bienenvolk zwei- oder dreimal behandelt, indem in die Futterstelle 0,5 bis 1,0 Liter alle 7 Tage eingegossen wird. Das schnellste und eindeutigste Ergebnis ist dann zu verzeichnen, wenn in dem von Varoatose befallenen Bienenvolk dieBrutflächen abgenommen werden oder wenn es überhaupt keine Brutflächen gibt.

Le A 22 737

- 8 -

Die Verbindung erwies sich auch als hocheffektiv in
bezug auf die Nabraulose (Läusebefall) bei Bienen. Die
Art und Weise der Anwendung entsprach der oben beschriebenen.


5    Vergleichsversuche:

| Mittel | Anzahl d. Bienenvölker | % d. Para-sitenbefalls | Abgefalle-Milben nach Behandlung | Abgefallene Milben nach Behandlung |
|---|---|---|---|---|
| erfindungs-gemäß | 10 | 8,5 % | 6290 | über 1000 |
| Stand der Technik | 10 | 6,4 % | 5406 | 800-880 |
| Kontrolle | 10 | 20,10 % | 373 | 46,50 |

Le A 22 737

- 9 -

Patentansprüche:

1) O,O-Diethyl-O-(3-chlor-4-methyl-cumarin-7-yl)-thiono-
   phosphorsäureester zur Bekämpfung von Varoa Jacobsoni
   auf Bienen.

2) O,O-Diethyl-O-(3-chlor-4-methyl-cumarin-7-yl)-thiono-
   phosphorsäureester zur Bekämpfung der Entwicklungsformen von Varoa Jacobsoni auf Bienen- und Drohnenlarven und Puppen.

3) Mittel zur Bekämpfung von Varoa Jacobson und deren
   Entwicklungsstadien auf Bienen, enthaltend O,O-Diethyl-
   O-(3-chlor-4-methyl-cumarin-7-yl)-thionophosphorsäure-
   ester, Trägersubstanzen und gegebenenfalls Formulierungshilfsmittel.

4) Mittel nach Anspruch 3, dadurch gekennzeichnet, daß
   es in der Anwendungsform 0,0000001 bis 95 Gew.-%
   O,O-Diethyl-O-(3-chlor-4-methyl-cumarin-7-yl)-thiono-
   phosphorsäureester enthält.

5) Mittel nach Anspruch 4, dadurch gekennzeichnet, daß
   es als Trägersubstanz Rohrzucker, Glukose oder einen
   anderen bienenverträglichen Zucker granuliert, kristallin, sirupös oder als wäßrige Lösung enthält.

6) Mittel nach Anspruch 3, dadurch gekennzeichnet, daß
   es auf 0,5 bis 1,0 Liter Zuckersirup 0,5 bis 0,6 g
   O,O-Diethyl-O-(3-chlor-4-methyl-cumarin-7-yl)-thiono-
   phosphorsäureester enthält.

7) Verfahren zur Herstellung von Mitteln nach Anspruch 3,
   dadurch gekennzeichnet, daß man einen Zuckersirup zubereitet, diesen auf 35 bis 40°C abkühlt, O,O-Diethyl-
   O-(3-chlor-4-methyl-cumarin-7-yl)-thionophosphorsäure-
   ester in Wasser suspendiert, diese Suspension dem
   Zuckersirup hinzufügt und gut vermischt.

Le A 22 737

8) Verwendung von O,O-Diethyl-O-(3-chlor-4-methyl-cumarin-7-yl)-thionophosphorsäureester zur und bei der Bekämpfung von Varoa Jacobsoni auf Bienen und deren Entwicklungsstadien auf Bienen- und Drohnenlarven und Puppen.

9) Verwendung von O,O-Diethyl-O-(3-chlor-4-methyl-cumarin-7-yl)-thionophosphorsäureester mit Zuckern als Trägersubstanz zur und bei der Bekämpfung von Varoa Jacobsoni auf Bienen und deren Entwicklungsstadien auf Bienen- und Drohnenlarven und Puppen.

10) Verwendung von O,O-Diethyl-O-(3-chlor-4-methyl-cumarin-7-yl)-thionophosphorsäureester zur und bei der Bekämpfung von Varoa Jacobsoni auf Bienen und deren Entwicklungsstadien auf Bienen- und Drohnenlarven und Puppen, dadurch gekennzeichnet, daß das parasitenbefallene Bienenvolk dreimal alle 7 Tage mit 0,5 bis 1,0 Liter Zuckersirup, in dem 0,5 bis 0,6 g der Verbindung O,O-Diethyl-O-(3-chlor-4-methyl-cumarin-7-yl)-thionophosphorsäureester befüttert wird.

Europäisches
Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-A-2 208 618 (BAYER) * Anspruch 1 * | 1 | A 01 N 57/16 |
| A | CHEMICAL ABSTRACTS Band 75, Nr. 11, 13. September 1971, Columbus, Ohio, USA; Seite 444, Spalte 1, Zusammenfassung Nr. 76528X T.F. KOZLOVA et al "Production of the insecticide preparation Coral (O.O-diethyl O-(3-chloro-4-methyl-7-coumarinyl ) thiophosphate). | 1 | |

RECHERCHIERTE SACHGEBIETE (Int. Cl.4)

A 01 N 57/16
A 61 K 31/665

Der vorliegende Recherchenbericht wurde für alle Patentansprüche erstellt.

| Recherchenort | Abschlußdatum der Recherche | Prüfer |
|---|---|---|
| BERLIN | 23-01-1985 | KAPTEYN H G |